# EUROPÄISCHE PATENTANMELDUNG

(11) **EP 0 557 834 A1**
(43) Veröffentlichungstag der Anmeldung: **01.09.1993**
(21) Anmeldenummer: 93102288.3
(22) Anmeldetag: 13.02.1993
(51) Int. Cl.: C07D 261/08, C07D 263/32, C07D 277/24, C07D 285/12

(54) **Verfahren zur Herstellung von heterocyclischen Aldehyden und Ketonen**

(30) Priorität: 28.02.1992 DE 4206170
(71) Anmelder: BASF Aktiengesellschaft, D-67063 Ludwigshafen (DE)
(72) Erfinder: Koenig, Hartmann, Dr., W-6703 Limburgerhof (DE); Goetz, Norbert, Dr., W-6520 Worms 1 (DE); Harreus, Albrecht, Dr., W-6700 Ludwigshafen (DE); Kirstgen, Reinhard, Dr., W-6730 Neustadt (DE); Oberdorf, Klaus, Dr., W-6900 Heidelberg (DE); Wingert, Horst, Dr., W-6800 Mannheim 1 (DE); Koehler, Ulrich, Dr., W-6800 Mannheim 51 (DE)

(57) **Zusammenfassung**

Herstellung von heterocyclischen Aldehyden und Ketonen der Formel I 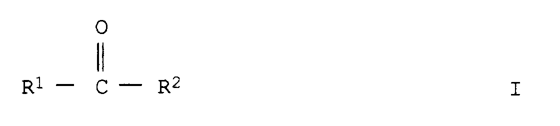
(R¹ = heterocyclischer Rest, R² = Wasserstoff oder Alkyl)
durch Oxidation der entsprechenden Halogenverbindung (II) 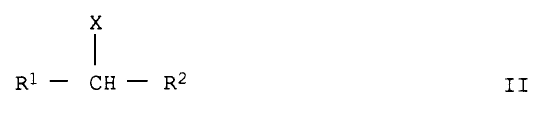
(X = Chlor, Brom oder Iod), indem man als Oxidationsmittel ein tertiäres Aminoxid (III) verwendet.

## Beschreibung

Die vorliegende Erfindung betrifft ein verbessertes Verfahren zur Herstellung von heterocyclischen Aldehyden und Ketonen der Formel I 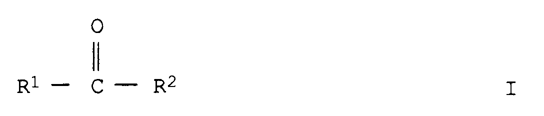
in der
- R¹: einen heterocyclischen Rest mit mindestens einem der Heteroatome Sauerstoff, Stickstoff und Schwefel und
- R²: Wasserstoff oder einen Alkylrest
bedeuten, durch Oxidation der entsprechenden Halogenverbindungen der Formel II 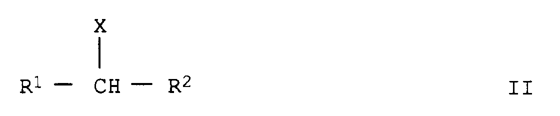
in der X Chlor, Brom oder Iod bedeutet.

Es ist allgemein bekannt, Aldehyde und Ketone durch Oxidation der entsprechenden primären oder sekundären Halogenverbindungen herzustellen

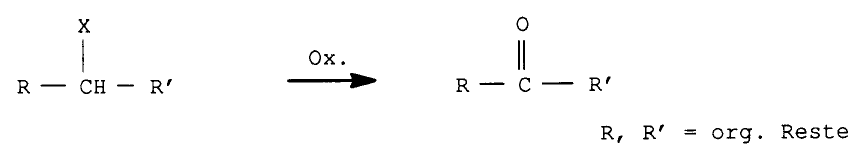

Als Oxidationsmittel verwendet man Dimethylsulfoxid, 2-Nitropropan/NaOEt, Hg(I)NO₃ oder K₂CrO₄ (J. March, Advanced Organic Chemistry, Seite 1081 bis 1083, Wiley and Sons, 3. Aufl., 1985). Jedoch sind die Ausbeuten und Selektivitäten meistens unbefriedigend, ganz abgesehen davon, daß die Verwendung dieser ökologisch und physiologisch nicht unbedenklichen Oxidationsmittel erhebliche verfahrenstechnische Probleme aufwirft.

In der gleichen Schrift wird das Oxidationsmittel Hexamethylentetramin genannt, welches für die Oxidation von primären Alkylhalogeniden zu Aldehyden Verwendung finden.

Aus Chem. Ber., 94, 2887 (1961) ist es bekannt, für die Herstellung von 2-Phenyl-4-formylthiazol aus dem entsprechenden 4-Halogenmethylthiazol Hexamethylentetramin zu verwenden.

Ferner wird in Arch. Pharmaz., Band 307, 81-88 (1974), die Oxidation weiterer Derivate des 2-Phenyl-4-halogenmethylthiazols mit Hexamethylentetramin beschrieben.

Weiterhin ist es aus der deutschen Patentschrift DE-C 29 48 058 bekannt, für die Oxidation von Alkyl- und Benzylhalogeniden zu den entsprechenden Carbonylverbindungen Aminoxide von tertiären Aminen oder von Pyridin einzuseten. So verwendet man beispielsweise im Falle der substituierten Benzaldehyde Trimethyl- und Triethylamin-N-oxid als Oxidationsmittel.

Über die Verwendung von 4-Dimethylaminopyridinoxid als Oxidationsagens für die Oxidation von 2-Chlormethylpyridin-Hydrochlorid zu 2-Formylpyridin berichten Mukaiyama et al (Bull. Chem. Soc. Jpn., 54, 2221 (1981)).

Der vorliegenden Erfindung lag daher die Aufgabe zugrunde, den geschilderten Mängeln abzuhelfen und heterocyclische Aldehyde und Ketone auf einfachere und wirtschaftlichere Weise zugänglich zu machen als bisher.

Demgemäß wurde ein Verfahren zur Herstellung von heterocyclischen Aldehyden und Ketonen der Formel I 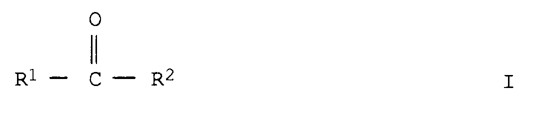
in der
- R¹: einen heterocyclischen Rest mit mindestens einem der Heteroatome Sauerstoff, Stickstoff und Schwefel und
- R²: Wasserstoff oder einen Alkylrest
bedeuten, durch Oxidation der entsprechenden Halogenverbindungen II 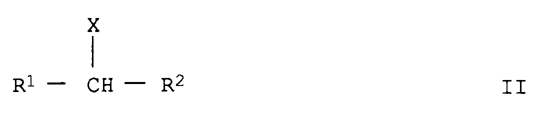
gefunden, welches dadurch gekennzeichnet ist, daß man hierzu als Oxidationsmittel ein tertiäres Aminoxid der Formel III 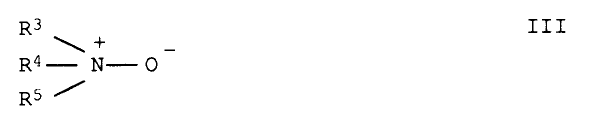
in der
- R³-R⁵: C-organische Reste, von denen zwei dieser Reste auch zu einem Ring verbunden sein können,
bedeuten, verwendet.

Ferner wurde gefunden, daß dieses Verfahren besonders gut gelingt, wenn man es zur Herstellung von heteroaromatischen Aldehyden der Formel Ia

R^{1a}-CHO Ia

in der R^{1a} einen 5- oder 6-gliedrigen heteroaromatischen Ring mit mindestens einem der Heteroatome Sauerstoff, Stickstoff und Schwefel bedeutet, aus den entsprechenden Halogenmethylverbindungen IIa

R^{1a}-CH₂-X IIa

anwendet.

Als Reste R¹ in den Verfahrensprodukten I bzw. Ausgangsverbindungen II eignen sich beispielsweise diejenigen, die sich von folgenden heterocyclischen Stammkörpern ableiten:

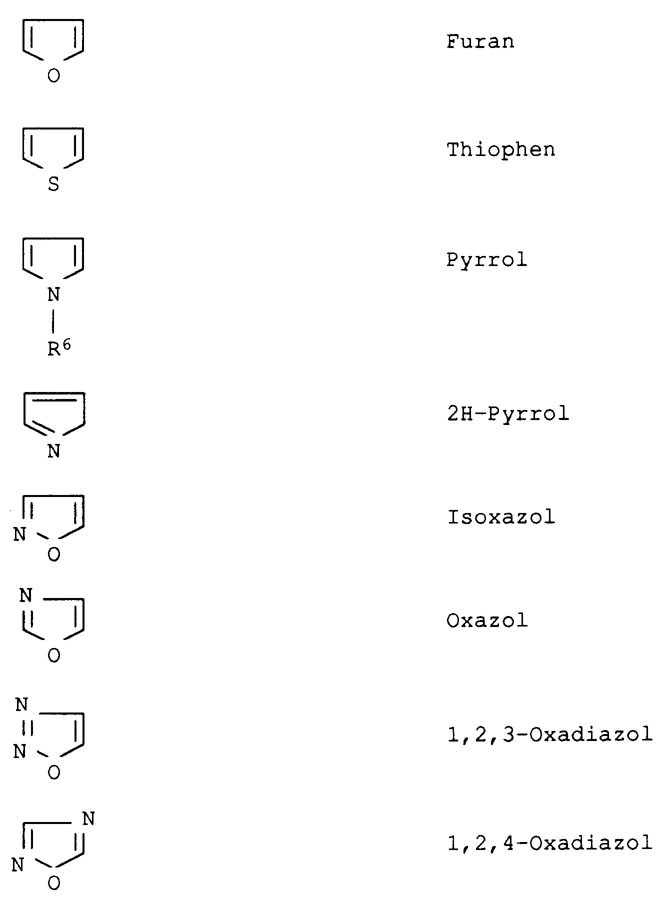

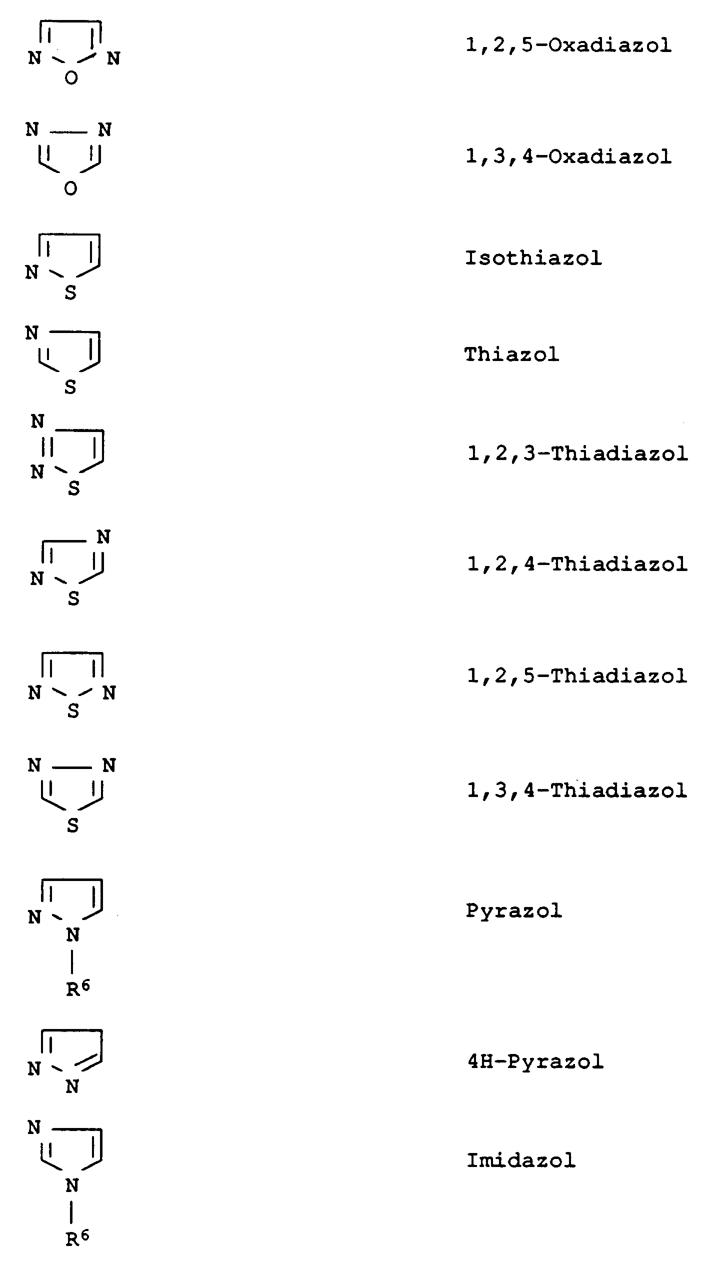

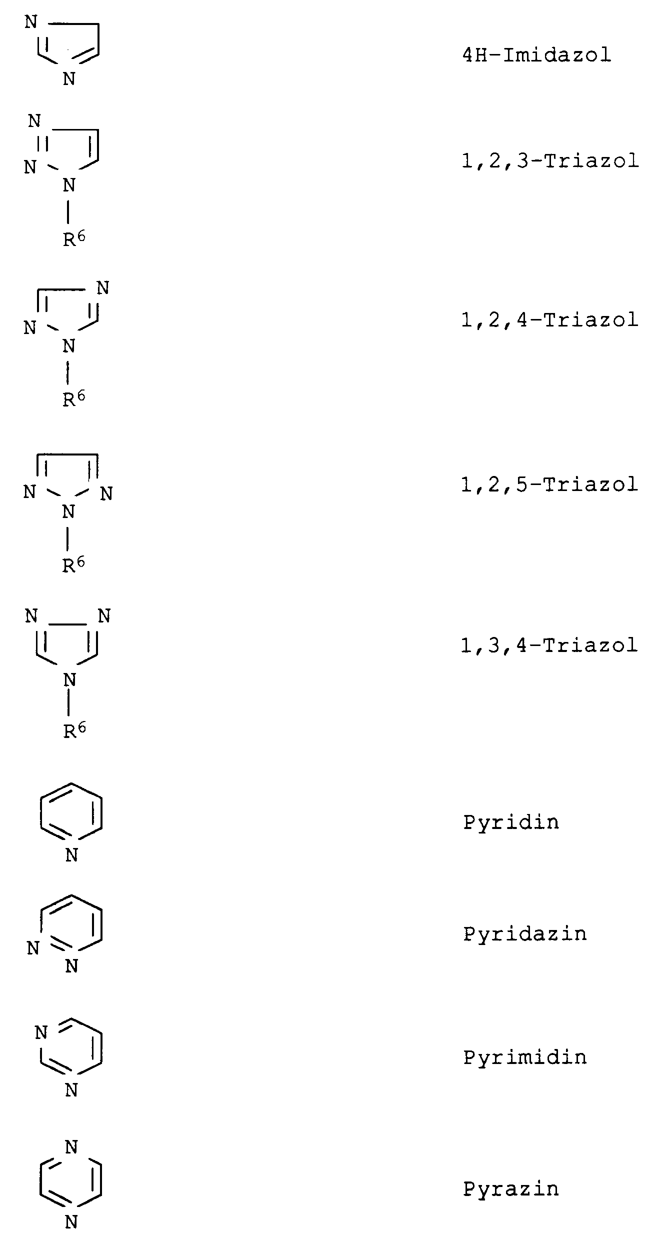

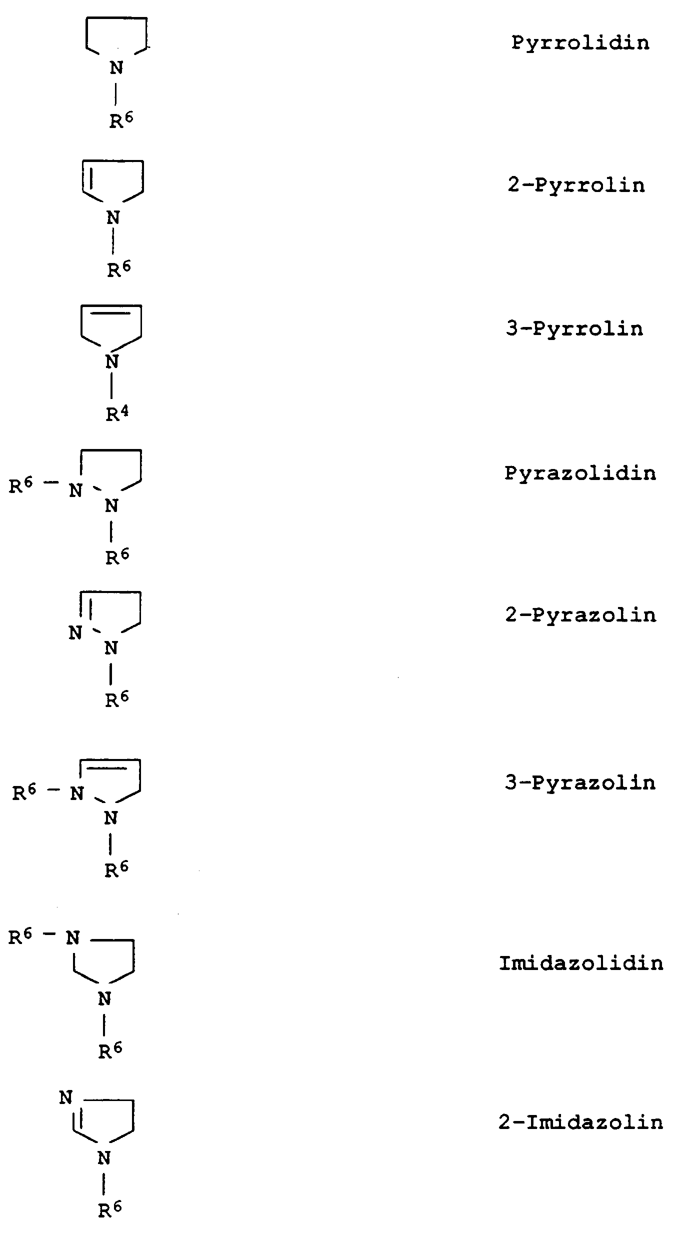

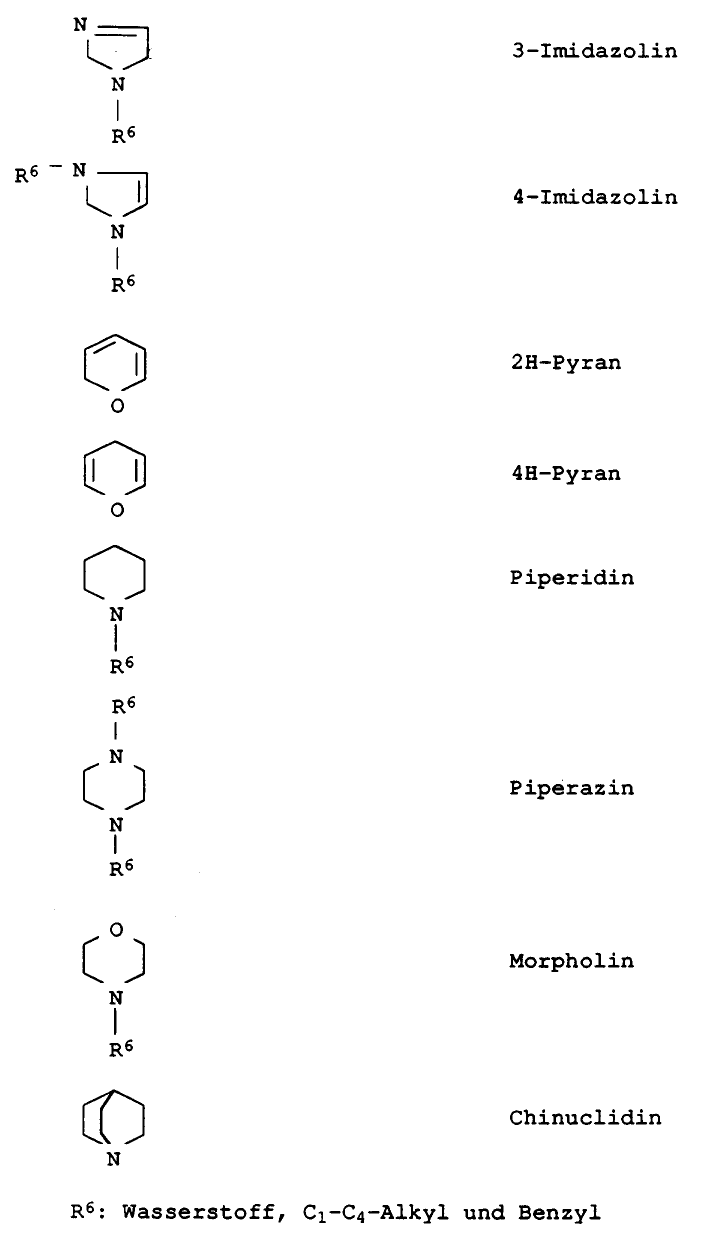

Bevorzugte Reste R¹ leiten sich von 5- und 6-gliedrigen heteroaromatischen Stammkörpern ab, wobei ganz besonders Isoxazol, Oxazol, Thiazol und 1,3,4-Thiadiazol in Betracht kommen.

Ferner können die Stammkörper auch anellierte Ringe tragen wie beispielsweise im
Benzofuran, Isobenzofuran, Benzothiophen, Indol, Isoindol, Benzisoxazol, Benzoxazol, Benzisothiazol, Benzthiazol, Indazol, Purin, Chinolin, Phthalazin, Chinoxalin, Indolin, Isoindolin, Chroman und im Isochroman.

In den entsprechenden Ausgangsverbindungen II kann die 1-Halogenalkylgruppe an ein beliebiges C-Atom des Ringes gebunden sein. Die übrigen C-Atome können beliebige inerte, also z.B. nicht selber oxidierbare organische, vorzugsweise über ein C-Atom gebundene Reste R'' tragen, beispielsweise
- Alkyl wie vor allem C₁-C₄-Alkyl wie Methyl, Ethyl, Propyl, Isopropyl, Butyl und Isobutyl;
- Cycloalkyl mit 3 bis 12 Ringgliedern wie Cyclopentyl und Cyclohexyl;
- cycloalkylsubstituiertes Alkyl wie 1- und 2-Cyclohexylethyl;
- Oxacycloalkyl mit 5 und 6 Ringgliedern wie 2- und 3-Oxacyclopentyl sowie 2-, 3- und 4-Oxacyclohexyl;
- Aryl wie Phenyl, α-Naphthyl und β-Naphthyl;
- Aralkyl wie Benzyl sowie 1- und 2-Phenylethyl;
- Alkoxy R''-O-, in dem R'' einen der vorgenannten Reste Alkyl, Cycloalkyl, cycloalkylsubstituiertes Alkyl, Aryl und Aralkyl bedeutet;
- Acyl R''-CO-, in dem R'' einen der sechs zuerst genannten Reste bedeutet; Beispiele für Acyl sind Acetyl und Benzoyl;
- Oxycarbonyl R''-O-CO-, in dem R'' einen der sechs zuerst genannten Reste bedeutet; Beispiele für Oxycarbonyl sind Methoxy- und Phenoxycarbonyl;
- Carbonyloxy R''-CO-O-, in dem R'' einer der sechs zuerst genannten Reste bedeutet.

Außerdem können die vorgenannten Reste durch C₁-C₄-Alkoxy und Cyano substituiert sein; die vorgenannten aromatischen Reste können weiterhin noch durch Fluor, Chlor und Brom substituiert sein.

R² kann Wasserstoff oder ein Alkylrest bedeuten.

Als Alkylreste kommen C₁-C₆-Alkyl wie Methyl, Ethyl, Propyl und Butyl, Cycloalkyl mit 3 bis 12 Ringgliedern wie Cyclopentyl und Cyclohexyl, cycloalkylsubstituiertes Alkyl wie 1- und 2-Cyclohexylethyl sowie Aralkyl wie Benzyl sowie 1- und 2-Phenylethyl in Betracht; sie können durch Cyano und Alkoxygruppen substituiert sein.

Die Ausgangsverbindungen II sind entweder bekannt oder können nach bekannten Methoden hergestellt werden. Entsprechende Herstellungsverfahren für die heteroaromatischen Halogenmethylverbindungen IIa sind z.B. beschrieben in: Comprehensive Heterocyclic Chemistry (Hrsg. A.R. Katritzky, C.W. Rees), 1. Auflage, Pergamon Press, Oxford, 1984, Bd. 2, S. 67 ff und S. 395 ff, Bd. 4 S. 313 ff und S. 657 ff, Bd. 5, S. 273 ff und S. 457 ff sowie Bd. 6, S. 61 ff.

Die im Hinblick auf die Verfahrensprodukte besonders wichtigen Ausgangsverbindungen IIa lassen sich beispielsweise wie folgt herstellen:

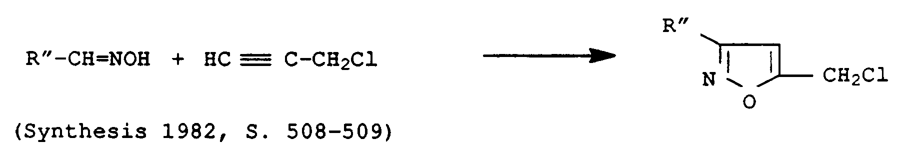

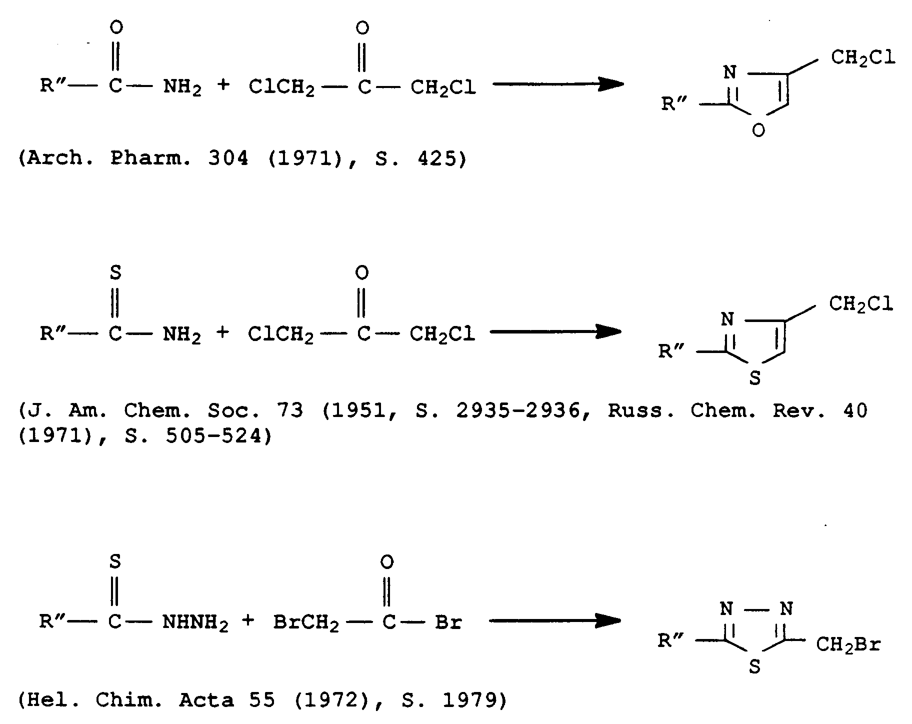

Als wichtige Verbindungen IIa, welche als Vorstufen der Zwischenprodukte Ia für die Synthese von Pflanzenschutzmitteln (s. z.B. EP-A 378 785 und DE-A 36 09 181) dienen, seien genannt:
5-Chlormethyl-3-isopropylisoxazol
4-Chlormethyl-2-(p-fluorphenyl)-oxazol
4-Chlormethyl-2-phenylthiazol
2-Brommethyl-5-(p-fluorphenyl)-1,3,4-thiadiazol
Als Oxidationsmittel dienen tertiäre Aminoxide (III), in der R³-R⁵ C-organische Reste, von denen zwei dieser Reste auch zu einem Ring verbunden sein können, bedeuten.

Die C-organischen Reste können aliphatische, cycloaliphatische und aromatische Reste sein, die außerdem durch Cyano, Nitro, Amino und Alkoxyruppen substituiert sein können.

Als Aminoxide mit aliphatischen Resten kommen beispielsweise die N-Oxide von
Trimethylamin
Triethylamin
Tripropylamin
Triisopropylamin
Tributylamin
Triisobutylamin
Diethylmethylamin
Dimethylethylamin und
Dimethylisopropylamin
in Betracht.

Als Aminoxide mit cycloaliphatischen Resten eignen sich beispielsweise die N-Oxide, die sich von folgenden Verbindungen ableiten:
Dimethylcyclopentylamin
Diethylcyclopentylamin
Dimethylcyclohexylamin und
Diethylcyclohexylamin.

Als Aminoxide mit aromatischen Resten seien beispielsweise die N-Oxide von
Dimethylanilin
Diethylanilin
Benzyldimethylamin
Benzyldiethylamin
p-Nitrophenyl-dimethylamin und
p-Nitrophenyl-diethylamin
genannt.

Als Aminoxide mit cycloaliphatischen Resten, die durch die Heteroatome Sauerstoff und Stickstoff unterbrochen sind, eignen sich die N-Oxide der
N-Alkyl- und N-Arylpiperidine
N-Alkyl- und N-Arylpiperazine
N-Alkyl- und N-Arylmorpholine und
N-Alkyl- und N-Arylazacycloheptane,
wobei sich in erster Linie die N-Alkyl- und N-Arylmorpholin-N-oxide eignen, darunter vor allem N-Methylmorpholin-N-oxid.

Die Aminoxide können sowohl in Form ihrer Hydrate als auch in Form ihrer wäßrigen Lösungen in unterschiedlichen Konzentrationen eingesetzt werden. Darüber hinaus ist es auch möglich, die Aminoxide im wasserfreien Zustand zu verwenden.

Für die vollständige Oxidation von II zu I sind mindestens äquimolare Mengen von III, bezogen auf die Menge von II, erforderlich. Im allgemeinen empfiehlt es sich jedoch, je Mol II 2 bis 5 mol, insbesondere 2,5 bis 3 mol Aminoxid III zu verwenden. Handelt es sich um sehr oxidationsempfindliche Verbindungen II oder I, kann es ratsam sein, den Umsatz durch Verwendung unterstöchiometrischer Mengen an III - etwa 0,8 bis 0,95 mol III pro Mol II - zu begrenzen.

Als organisches Reaktionsmedium kommen Flüssigkeiten in Betracht, in denen die Ausgangsstoffe löslich sind, z.B. aliphatische und aromatische Kohlenwasserstoffe wie Hexan, Heptan, Cyclohexan, Benzol, Toluol, Xylole, chlorierte und nitrierte Kohlenwasserstoffe wie Chloroform, Dichlormethan, 1,2-Dichlorethan, Tetrachlorkohlenstoff, Chlorbenzol, Dichlorbenzol und Nitrobenzol, aliphatische Alkohole wie Methanol, Ethanol und Isopropanol, Carbonsäureester wie Essigsäure- und Propionsäurealkylester, Alkylcyanide wie Acetonitril, Sulfoxide wie Dimethylsulfoxid und Sulfone wie Sulfolan sowie Mischungen hiervon. Die Umsetzung ist auch durchführbar, wenn nur Verbindung II im Reaktionsmedium gelöst ist, und das Aminoxid III in suspendierter Form vorliegt.

Bei Verwendung des Aminoxids in Form einer wäßrigen Lösung ist die Mischbarkeit von organischem Lösungsmittel und wäßriger Phase nicht erforderlich, so daß die Umsetzung auch in einem ständig durchrührten Zweiphasengemisch abläuft.

In der Regel ist es zweckmäßig, daß die Reaktion in einer Menge von 1 bis 10 kg Lösungsmittel pro kg II durchgeführt wird.

Die Reaktionstemperatur liegt im allgemeinen zwischen 20 und 150°C, vorzugsweise zwischen 20 und 80°C, und ist durch die Siedetemperatur des Lösungsmittels begrenzt. Es empfiehlt sich, die Umsetzung bei der Siedetemperatur des Lösungsmittels durchzuführen. Es kann auch ein höher als die Reaktionstemperatur siedendes Lösungsmittel gewählt werden, sofern der angegebene Temperaturbereich eingehalten wird.

Im allgemeinen arbeitet man unter Atmosphärendruck.

Die Durchführung des erfindungsgemäßen Verfahrens, welches nach den üblichen Techniken sowohl diskontinuierlich als auch kontinuierlich gestaltet werden kann, erfolgt auf an sich bekannte Weise und weist keine verfahrenstechnischen Besonderheiten auf, so daß nähere Angaben hierzu entbehrlich sind. Das gleiche gilt für die Aufarbeitung der Reaktionsgemische, bei der man die Verfahrensprodukte wie üblich aus der organischen Phase isoliert. Die zurückbleibende wäßrige Phase, in der sich nicht umgesetztes Aminoxid und das entsprechende tertiäre Amin befinden, kann aufgearbeitet werden. Das tertiäre Amin kann beispielsweise durch Wasserstoffperoxid - wie in der Patentschrift DE-C 1 694 048 beschrieben - wieder in das jeweilige Aminoxid überführt werden.

Man erhält die heterocyclischen Aldehyde und Ketone, die u.a. wertvolle Zwischenprodukte für die Herstellung von Pflanzenschutzmitteln sind, in unerwartet hohen Ausbeuten und hoher Reinheit.

### Beispiel 1

### Herstellung von 5-Formyl-3-isopropylisoxazol

8 g (50 mmol) 5-Chlormethyl-3-isopropylisoxazol wurden in 40 ml Toluol gelöst und mit 20,3 g (150 mmol) N-Methylmorpholin-N-oxid-Monohydrat versetzt. Die Mischung wurde 7 Stunden auf 85°C erhitzt, nach Abkühlung mit Wasser versetzt und mehrmals mit Toluol extrahiert. Nach der destillativen Entfernung des Lösungsmittels aus den vereinigten organischen Phasen wurde das Produkt in einer Ausbeute von 70 % erhalten.

### Beispiel 2

### Herstellung von 4-Formyl-2-(p-fluorphenyl)-oxazol

2,5 g (12 mmol) 4-Chlormethyl-2-(p-fluorphenyl)-oxazol wurden in 15 ml Dimethylsulfoxid gelöst, mit 8,1 g (60 mmol) N-Methylmorpholin-N-oxid-Monohydrat versetzt und 24 Stunden bei Raumtemperatur gerührt. Nach Eingießen der Reaktionsmischung in Wasser wurde die organische Phase abgetrennt, und die wäßrige wurde mehrmals mit Essigester extrahiert. Aus den vereinigten organischen Phasen wurde nach Aufarbeitung das Produkt in einer Ausbeute von 48 % erhalten.

### Beispiel 3

### Herstellung von 4-Formyl-2-phenylthiazol

10,5 g (50 mmol) 4-Chlormethyl-2-phenylthiazol wurden in 50 ml Essigsäureethylester gelöst, mit 33,8 g (250 mmol) N-Methylmorpholin-N-oxid-Monohydrat versetzt und 5 Stunden bei Rückfluß erhitzt. Die Aufarbeitung erfolgte nach Beispiel 2. Das Produkt wurde in einer Ausbeute von 82 % erhalten.

### Beispiel 4

### Herstellung von 5-Formyl-2-(p-fluorphenyl)-1,3,4-thiadiazol

5 g (18 mmol) 2-Brommethyl-5-(p-fluorphenyl)-1,3,4-thiadiazol wurden in 25 ml Dimethylsulfoxid gelöst, mit 12,4 g (92 mmol) N-Methylmorpholin-N-oxid-Monohydrat versetzt und 2 Stunden bei Raumtemperatur gerührt. Nach Eingießen der Reaktionsmischung in Wasser wurde die organische Phase abgetrennt, und die wäßrige wurde mit Methyl-tert.-butylether extrahiert. Aus den vereinigten organischen Phasen wurde nach Aufarbeitung das Produkt in einer Ausbeute von 54 % erhalten.

## Patentansprüche

1. Verfahren zur Herstellung von heterocyclischen Aldehyden und Ketonen der Formel I 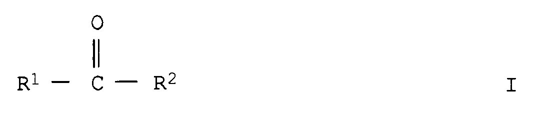 in der
R¹ einen heterocyclischen Rest mit mindestens einem der Heteroatome Sauerstoff, Stickstoff und Schwefel und
R² Wasserstoff oder einen Alkylrest
bedeuten, durch Oxidation der entsprechenden Halogenverbindungen der Formel II 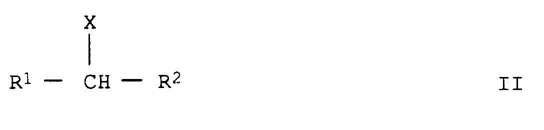 in der
X Chlor, Brom oder Iod
bedeutet, dadurch gekennzeichnet, daß man als Oxidationsmittel ein tertiäres Aminoxid der Formel III 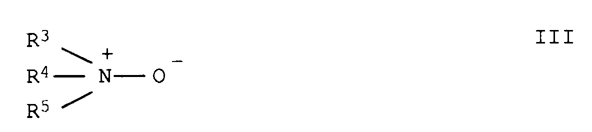 in der
R³-R⁵ C-organische Reste, von denen zwei dieser Reste auch zu einem Ring verbunden sein können,
bedeuten, verwendet.

2. Verfahren nach Anspruch 1, dadurch gekennzeichnet, daß man als Oxidationsmittel ein N-substituiertes Morpholinoxid der Formel IIIa 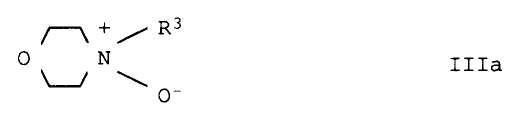 verwendet.

3. Verfahren nach Anspruch 1 oder 2, dadurch gekennzeichnet, daß man es zur Herstellung von heteroaromatischen Aldehyden der Formel Ia
R^{1a}-CHO Ia
in der
R^{1a} einen 5- oder 6-gliedrigen heteroaromatischen Ring mit mindestens einem der Heteroatome Sauerstoff, Stickstoff und Schwefel
bedeutet, aus den entsprechenden Halogenmethylverbindungen IIa
R^{1a}-CH₂-X IIa
anwendet.
